# EUROPEAN PATENT APPLICATION

(11) **EP 4 316 479 A1**
(43) Date of publication of application: **07.02.2024**
(21) Application number: 21933242.6
(22) Date of filing: 03.12.2021
(51) Int. Cl.: A61K 31/197, A61K 33/34, A61K 33/06, A61K 33/24, A61K 33/26, A61K 33/30, A61P 43/00, A61P 31/12

(54) **PHARMACEUTICAL COMPOSITION FOR SUPPRESSING TMPRSS2 EXPRESSION**

(30) Priority: 26.03.2021 JP 2021052802
(71) Applicant: Tokyo Institute of Technology, Tokyo 152-8550 (JP); SBI Pharmaceuticals Co., Ltd., Tokyo 106-6020 (JP)
(72) Inventor: OGURA, Shunichiro, Tokyo 152-8550 (JP); NAKAJIMA, Motowo, Tokyo 106-6020 (JP); ISHIZUKA, Masahiro, Tokyo 106-6020 (JP); ISHII, Takuya, Tokyo 106-6020 (JP); IMAZATO, Hideo, Tokyo 106-6020 (JP)
(74) Representative: Weickmann & Weickmann PartmbB
(86) International application number: PCT/JP2021/044426
(87) International publication number: WO 2022/201644

(57) **Abstract**

The object of the present invention is to search for a compound that may suppress the expression of TMPRSS2 in a cell or tissue. The expression of TMPRSS2 in a cell or tissue is suppressed by 5-aminolevulinic acids.

## Description

### Technical Field

The present invention relates to a pharmaceutical composition for suppressing the expression of type II transmembrane serine protease (TMPRSS2) in a cell or tissue, or a method for suppressing the expression of TMPRSS2 in a cell or tissue.

### Background Art

Membrane fusion proteins of many respiratory viruses are synthesized as precursors without fusion activity, which become active (cleavage activation) by being appropriately cleaved by the action of particular protein degradation enzymes (proteases). In recent years, it has become clear that one of the major proteases that activate respiratory viruses in the respiratory tract is TMPRSS2 (e.g., Non-Patent Literature 1).

### Citation List

[Non-Patent Literature 1] Sakai K, et al., J Virol 88: 5608-5616, 2014

### Summary of the Invention

### Problems to be Solved by the Invention

The present inventors searched for a compound that may suppress the expression of TMPRSS2 in a cell or tissue.

### Means for Solving the Problems

Surprisingly, the present inventors found that 5-aminolevulinic acids (ALAs) has the effect of suppressing the expression of TMPRSS2 in a cell or tissue.

In other words, in one embodiment, the present invention relates to a pharmaceutical composition for suppressing the expression of TMPRSS2, comprising a compound shown by the following Formula (I):
[Chemical Formula 1]

R¹-NHCH₂COCH₂CH₂COOR² (I)

(wherein R¹ represents a hydrogen atom or an acyl group, and R² represents a hydrogen atom, a linear or branched alkyl group, a cycloalkyl group, an aryl group, or an aralkyl group)
or a salt or an ester thereof.

One embodiment of the present invention is characterized in that said pharmaceutical composition further suppress the expression of angiotensin converting enzyme 2 (ACE2) in addition to said TMPRSS2.

One embodiment of the present invention is characterized in that R¹ is selected from the group consisting of a hydrogen atom, an alkanoyl group having 1-8 carbons, and an aroyl group having 7 - 14 carbons, and

R² is selected from the group consisting of a hydrogen atom, a linear or branched alkyl group having 1-8 carbons, a cycloalkyl group having 3-8 carbons, an aryl group having 6-14 carbons, and an aralkyl group having 7 - 15 carbons.

One embodiment of the present invention is characterized in that R¹ and R² are hydrogen atoms.

One embodiment of the present invention is characterized in that the pharmaceutical composition further contains one or two or more types of metal-containing compounds.

One embodiment of the present invention is characterized in that the metal-containing compound is a compound containing iron, magnesium, zinc, nickel, vanadium, copper, chromium, molybdenum, or cobalt.

One embodiment of the present invention is characterized in that the metal-containing compound is a compound containing iron.

One embodiment of the present invention is characterized in that said compound containing iron is sodium ferrous citrate.

One embodiment of the present invention is characterized in that the pharmaceutical composition is a pharmaceutical composition for use in prevention or treatment of a disease related to expression of TMPRSS2 or ACE2 in a cell or tissue.

One embodiment of the present invention is characterized in that said disease related to expression of TMPRSS2 or ACE2 is an infection by a virus that infects by utilizing TMPRSS2 or ACE2.

Another embodiment of the present invention relates to the use of a compound shown by the following Formula (I):
[Chemical Formula 2]

R¹-NHCH₂COCH₂CH₂COOR² (I)

(wherein R¹ represents a hydrogen atom or an acyl group, and R² represents a hydrogen atom, a linear or branched alkyl group, a cycloalkyl group, an aryl group, or an aralkyl group)
or a salt or an ester thereof, in manufacturing a medication for suppressing the expression of TMPRSS2.

Another embodiment of the present invention relates to a method for suppressing the expression of TMPRSS2, comprising a step of applying to a subject an effective amount of a compound shown by the following Formula (I):
[Chemical Formula 3]

R¹-NHCH₂COCH₂CH₂COOR₂ (I)

(wherein R¹ represents a hydrogen atom or an acyl group, and R² represents a hydrogen atom, a linear or branched alkyl group, a cycloalkyl group, an aryl group, or an aralkyl group)
or a salt or an ester thereof.

Another embodiment of the present invention relates to a kit for suppressing the expression of TMPRSS2, characterized in that it comprises a compound shown by the following Formula (I): 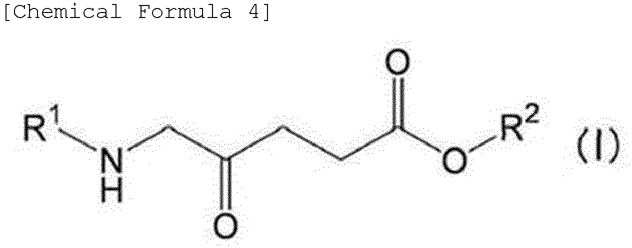 (wherein R¹ represents a hydrogen atom or an acyl group, and R² represents a hydrogen atom, a linear or branched alkyl group, a cycloalkyl group, an aryl group, or an aralkyl group)
or a pharmaceutically acceptable salt or ester thereof.

One embodiment of the present invention is characterized in that said kit further comprises a description comprising instructions on the dosage and the administration method for the compound shown by said Formula (I).

One embodiment of the present invention is characterized in that said kit further comprises a description comprising instructions on the dosage and the administration method for the compound shown by said Formula (I) and the metal-containing compound.

Note that an invention of any combination of one or more characteristics of the present invention described above is also encompassed by the scope of the present invention.

### Brief Description of the Drawings

[Figure 1] Figure 1 shows that the amount of ACE2 protein expressed in a cell was significantly reduced by application of ALA.
[Figure 2] Figure 2 shows that the amount of ACE2 protein expressed in a cell was reduced by application of ALA or ALA + SFC.
[Figure 3] Figure 3 shows the change in the amounts of ACE2 and TMPRSS2 genes expressed in the nasal mucosa after administering 5-ALA (300 mg/body) + SFC (334 mg/body) to test subjects.
[Figure 4] Figure 4 shows the change in the amounts of ACE2 and TMPRSS2 genes expressed in the nasal mucosa after administering 5-ALA (100 mg/body) + SFC (115 mg/body) to test subjects.

### Description of Embodiments

ALAs herein is ALA or derivatives thereof, or salts thereof.

ALA herein means 5-aminolevulinic acid. ALA is also referred to as δ-aminolevulinic acid, and it is a type of amino acid.

Compounds represented by the following Formula (I) can be exemplified as derivatives of ALA. In Formula (I), R¹ represents a hydrogen atom or an acyl group, and R² represents a hydrogen atom, a linear or branched alkyl group, a cycloalkyl group, an aryl group, or an aralkyl group. Note that in Formula (I), ALA corresponds to the case where R¹ and R² are hydrogen atoms.
[Chemical Formula 5]

R¹-NHCH₂COCH₂CH₂COOR² (I)

ALAs may act as an active ingredient in the state of ALA of Formula (I) or a derivative thereof in the body, and it can also be administered as a prodrug (precursor) that is degraded by an enzyme in the body.

The acyl group in R¹ of Formula (I) can include a linear or branched alkanoyl group having 1-8 carbons, such as formyl, acetyl, propionyl, butyryl, isobutyryl, valeryl, isovaleryl, pivaloyl, hexanoyl, octanoyl, and benzylcarbonyl groups, and an aroyl group having 7-14 carbons, such as benzoyl, 1-naphthoyl, and 2-naphthoyl groups.

The alkyl group in R² of Formula (I) can include a linear or branched alkyl group having 1-8 carbons, such as methyl, ethyl, propyl, isopropyl, butyl, isobutyl, sec-butyl, tert-butyl, pentyl, isopentyl, neopentyl, hexyl, heptyl, and octyl groups.

The cycloalkyl group in R² of Formula (I) can include a cycloalkyl group having 3 - 8 carbons which may have saturated or partially unsaturated bonds present, such as cyclopropyl, cyclobutyl, cyclopentyl, cyclohexyl, cycloheptyl, cyclooctyl, cyclododecyl, and 1-cyclohexenyl groups.

The aryl group in R² of Formula (I) can include an aryl group having 6 - 14 carbons, such as phenyl, naphthyl, anthryl, and phenanthryl groups.

For the aralkyl group in R² of Formula (I), the aryl moiety can be equally exemplified by the above aryl group and the alkyl moiety can be equally exemplified by the above alkyl group, and can specifically include an aralkyl group having 7 - 15 carbons, such as benzyl, phenethyl, phenylpropyl, phenylbutyl, benzhydryl, trityl, naphthylmethyl, and naphthylethyl groups.

Preferred ALA derivatives include compounds where R¹ is formyl, acetyl, propionyl, and butyryl groups, etc. Moreover, preferred ALA derivatives include compounds where the above R² is methyl, ethyl, propyl, butyl, and pentyl groups. Moreover, preferred ALA derivatives include compounds where the combination of the above R¹ and R² is each combination of (formyl and methyl), (acetyl and methyl), (propionyl and methyl), (butyryl and methyl), (formyl and ethyl), (acetyl and ethyl), (propionyl and ethyl), and (butyryl and ethyl).

Among ALAs, a salt of ALA or a derivative thereof can include a pharmaceutically acceptable acid addition salt, a metal salt, an ammonium salt, and an organic amine addition salt, etc. Exemplification of acid addition salts can include, e.g., each of inorganic acid salts such as a hydrochloride salt, a hydrobromide salt, a hydroiodide salt, a phosphate salt, a nitrate salt, and a sulfate salt, as well as each of organic acid addition salts such as a formate salt, an acetate salt, a propionate salt, a toluenesulfate salt, a succinate salt, an oxalate salt, a lactate salt, a tartrate salt, a glycolate salt, a methanesulfonate salt, a butyrate salt, a valerate salt, a citrate salt, a fumarate salt, a maleate salt, and a malate salt. Exemplification of metal salts can include each of alkali metal salts such as a lithium salt, a sodium salt, and a potassium salt, each of alkaline earth metal salts such as magnesium and calcium salts, as well as each of metal salts such as aluminum and zinc. Exemplification of ammonium salts can include alkyl ammonium salts such an ammonium salt and a tetramethylammonium salt, etc. Exemplification of organic amine salts can include each salt of a triethylamine salt, a piperidine salt, a morpholine salt, and a toluidine salt, etc. Note that these salts may also be employed as a solution at the time of use.

Among the above ALAs, the most desirable are ALA, and various esters such as ALA methyl ester, ALA ethyl ester, ALA propyl ester, ALA butyl ester, and ALA pentyl ester, etc., as well as hydrochloride salts, phosphate salts, and sulfate salts thereof. Among these, ALA hydrochloride salt and ALA phosphate salt can be exemplified as particularly favorable.

The above ALAs can be manufactured by well-known methods such as e.g. chemical synthesis, production by microorganisms, and production by enzymes. Moreover, the above ALAs may form hydrates or solvates, and ALAs may be employed alone or in an appropriate combination of two or more.

When preparing the above ALAs as an aqueous solution, in order to prevent degradation of ALAs, care must be taken so that the aqueous solution will not become alkaline. If it becomes alkaline, degradation can be prevented by removing oxygen.

The optimal amount for the dosage of ALAs to the subject may be determined by those skilled in the art (such as a physician) according to the purpose of administration. Specifically, by ALA conversion, the dosage may be 0.1 mg - 1000 mg/day, 0.2 mg - 500 mg/day, 0.3 mg - 250 mg/day, 0.4 mg - 100 mg/day, or 0.5 mg - 20 mg/day per 1 kg of subject body weight.

In one embodiment, the pharmaceutical composition of the present invention may further contain one or two or more types of metal-containing compounds. The metal moiety of such metal-containing compounds can include iron, magnesium, zinc, nickel, vanadium, cobalt, copper, chromium, and molybdenum. In a preferred embodiment, the metal moiety of the metal-containing compound is preferably iron, magnesium, or zinc, particularly iron.

Iron compounds that may be employed in the present invention may be an organic salt or an inorganic salt. Inorganic salts can include ferric chloride, iron sesquioxide, iron sulfate, and ferrous pyrophosphate. Organic salts can include organic acid salts, such as a carboxylic salt such as hydroxycarboxylic salt, a citrate salt such as ferrous citrate, iron sodium citrate, sodium ferrous citrate (SFC), and iron ammonium citrate, as well as ferric pyrophosphate, heme iron, iron dextran, iron lactate, ferrous gluconate, iron sodium diethylenetriaminepentaacetate, iron ammonium diethylenetriaminepentaacetate, iron sodium ethylenediaminetetraacetate, iron ammonium ethylenediaminepentaacetate, iron sodium dicarboxymethylglutamate, iron ammonium dicarboxymethylglutamate, ferrous fumarate, iron acetate, iron oxalate, ferrous succinate, and iron sodium succinate citrate, an well as triethylenetetramine iron, lactoferrin iron, transferrin iron, sodium iron chlorophyllin, ferritin iron, saccharated iron oxide, and ferrous glycine sulfate.

Magnesium compounds that may be employed in the present invention can include magnesium citrate, magnesium benzoate, magnesium acetate, magnesium oxide, magnesium chloride, magnesium hydroxide, magnesium carbonate, magnesium sulfate, magnesium silicate, magnesium nitrate, magnesium diammonium diethylenetriaminepentaacetate, magnesium disodium ethylenediaminetetraacetate, and magnesium protoporphyrin.

Zinc compounds that may be employed in the present invention can include zinc chloride, zinc oxide, zinc nitrate, zinc carbonate, zinc sulfate, zinc diammonium diethylenetriaminepentaacetate, zinc disodium ethylenediaminetetraacetate, zinc protoporphyrin, and zinc-containing yeast.

The dosage of the metal-containing compound to the subject may be 0 - 100 folds in molar ratio to the dosage of ALA to the subject, desirably 0.01 folds - 10 folds, more desirably 0.05 folds - 5 folds, and further desirably 0.125 - 1.0 folds.

ALAs and the metal-containing compound contained in the pharmaceutical composition of the present invention can be administered as a composition comprising ALAs and the metal-containing compound or also as each alone, although even when administering each alone, it is preferred to administer them simultaneously. Simultaneously herein means not only performing at the same time, but also, even if not at the same time, performing the administration of ALAs and the metal-containing compound without considerable interval between the two so that an additive effect, preferably a synergistic effect can take effect.

The administration route of ALAs and the metal-containing compound in the present invention is not limited, and may be systemic administration or local administration. The administration route can include, e.g., oral administration including sublingual administration, or inhalation administration, direct administration to target tissue or organ by a catheter, intravenous administration including infusion, transdermal administration by e.g. a patch, suppository, or parenteral administration such as administration by forced enteral nutrition employing a nasogastric tube, a nasointestinal tube, a gastrostomy tube, or an enterostomy tube, and the like, although oral administration is particularly preferred. Moreover, as described above, ALAs and the metal-containing compound may also be administered from separate routes.

The dosage form of ALAs, a metal-containing compound, or a combination drug of a combination thereof employed in the present invention may be appropriately determined according to said administration route, and can include, but is not limited to, injections, infusions, tablets, capsules, fine granules, powders, liquids, liquors dissolved in syrups etc., patches, suppositories, and the like.

The pharmaceutical composition according to the present invention can have other optional ingredients such as medicinal ingredients, nutrients, supports added thereto as necessary. As optional ingredients, for example, various drug compounding ingredients such as ordinary pharmaceutically acceptable supports, e.g. crystalline cellulose, gelatin, lactose, starch, magnesium stearate, talc, plant and animal fat, fat and oil, gum, and polyalkylene glycol, etc., binders, stabilizers, solvents, dispersion media, expanders, excipients, diluents, pH buffers, disintegrants, solubilizers, solubilizing agents, isotonic agents, and the like can be added.

According to the present invention, expression of TMPRSS2 in a cell or tissue can be suppressed by application of ALAs. TMPRSS2 is a type of type II transmembrane serine protease, and is primarily expressed in the prostate gland or the respiratory tract epithelium. Although the physiological significance of TMPRSS2 is unidentified, it has become clear that one of the major proteases that activate respiratory viruses in the respiratory tract is TMPRSS2 (Sakai K, et al., J Virol 88: 5608-5616, 2014). Accordingly, the cells or tissues to be the target for the present invention may be the respiratory tract epithelium (such as epithelial cells or epithelial tissues of nare, nasal cavity, pharynx, larynx, trachea, bronchial tube, and pulmonary alveolus). Moreover, the cells or tissues to be the target for the present invention further include oral cavity, tongue, taste buds, and gingival sulcus etc.

The present invention can be employed in the prevention or treatment of a disease related to expression of TMPRSS2 by suppressing the expression of TMPRSS2 in a cell or tissue. As described above, since TMPRSS2 is one of the major proteases that activate respiratory viruses in the respiratory tract, the present invention can be employed in the prevention or treatment of a respiratory virus infection. The respiratory viruses to be the target for the present invention may be, but are not limited to, e. g. , viruses that utilize TMPRSS2 in the infection route to humans or animals (such as influenza virus, human metapneumovirus, human parainfluenza virus, and coronavirus (SARS-CoV, SARS-CoV-2, Human Coronavirus-NL63)).

When the present invention is employed for the prevention or treatment of an infection by a virus that infects by utilizing TMPRSS2, it may be used in combination with one or two or more other agents (such as vaccines or therapeutic drugs for viruses exemplified above). The dosage, administration route, etc. of the other agents to the subject is not limited, and for example can be appropriately determined by those skilled in the art (such as a physician) according to the package insert of the agent.

Note that when referring to "suppressing the expression of TMPRSS2 in a cell or tissue" herein, it may mean suppressing or inhibiting at least a part of the process in which the TMPRSS2 protein is expressed from the gene encoding TMPRSS2, or it may also mean reducing the amount of TMPRSS2 protein already expressed.

According to the present invention, expression of ACE2 in a cell or tissue can be suppressed by application of ALAs. Since ACE2 is known to be universally expressed in most tissues throughout the body, the cells or tissues to be the target for the present invention may be various cells or tissues.

The present invention can be employed in the prevention or treatment of a disease related to expression of ACE2 by suppressing the expression of ACE2 in a cell or tissue. Since ACE2 is known for the conversion of angiotensin II to angiotensin-(1-7) in the body to suppress the renin/angiotensin/aldosterone system related to blood pressure control, the present invention can be employed in the prevention or treatment of e.g. a metabolic disease related to expression of ACE2.

Note that when referring to "suppressing the expression of ACE2 in a cell or tissue" herein, it may mean suppressing or inhibiting at least a part of the process in which the ACE2 protein is expressed from the gene encoding ACE2, or it may also mean reducing the amount of ACE2 protein already expressed.

Moreover, non-limiting examples of diseases related to expression of ACE2 can include infection by viruses that infect via ACE2 (such as SARS-CoV, SARS-CoV-2, Human Coronavirus-NL63) . SARS-CoV (Severe acute respiratory syndrome coronavirus) herein may mean a virus recognized from the taxonomical viewpoint to be the same viral species as the virus identified as the pathogen of severe acute respiratory syndrome (SARS) that was epidemic in 2003 in China etc. (such as a virus classified as such by the International Committee on Taxonomy of Viruses). Moreover, SARS-CoV-2 (Severe acute respiratory syndrome coronavirus 2) herein may mean a virus taxonomically recognized to be the same viral species as the virus identified as the pathogen of the new variant coronavirus infection (COVID-19) that was epidemic throughout the world from 2019 (such as a virus classified as such by the International Committee on Taxonomy of Viruses).

When the present invention is employed for the prevention or treatment of an infection by a virus that infects via ACE2, it may be used in combination with one or two or more other agents (such as vaccines or therapeutic drugs for SARS-CoV or SARS-CoV-2). The dosage, administration route, etc. of the other agents to the subject is not limited, and for example can be appropriately determined by those skilled in the art (such as a physician) according to the package insert of the agent.

The terms used herein, except for those that are particularly defined, are employed for describing particular embodiments, and do not intend to limit the invention.

Moreover, the term "comprising" as used herein, unless the content clearly indicates to be understood otherwise, intends the presence of the described items (such as components, steps, elements, and numbers), and does not exclude the presence of other items (such as components, steps, elements, and numbers).

Unless otherwise defined, all terms used herein (including technical and scientific terms) have the same meanings as those broadly recognized by those skilled in the art of the technology to which the present invention belongs. The terms used herein, unless explicitly defined otherwise, are to be construed as having meanings consistent with the meanings herein and in related technical fields, and shall not be construed as having idealized or excessively formal meanings.

The present invention will now be described in further detail with reference to Examples. However, the present invention can be embodied by various aspects, and shall not be construed as being limited to the Examples described herein.

### Examples

### [Experiment 1: Effect of ALA on amount of ACE2 protein expressed]

(i) Human liver cancer-derived cell strain HepG2 was cultured for 2 days with Dulbecco's modified Eagle medium (DMEM) comprising 10% fetal bovine serum (FBS) under 5% CO₂.
(ii) After 2 days, 5-aminolevulinic acid hydrochloride salt (ALA) was supplemented to the DMEM comprising FBS to a final concentration of 1 mM, and the cells of (i) were cultured in said medium at 37°C for 48 hours. Note that those without any supplements was set as the control.
(iii) After 48 hours, the cell supernatant was discarded, the cells were washed with PBS, and then Lysis buffer A (+) (50 mM Tris-HCl (pH 7.4), 20 mM N-methylmaleimide, 1 mM DTT, 1 % (v/v) Triton X-100, and Protease inhibitor cocktail) was added, and the cell lysate was collected. Homogenization and centrifugation (3,000 rpm, 10 minutes, 4°C) were then performed, and the residue was removed. A part of the cell lysate collected was quantified for proteins with the Bradford method, and the remainder was mixed with SDS sample buffer (50 mM Tris-HCl, pH 6. 8, 2% SDS, 2.5% mercaptoethanol, 10% glycerol, 0.01% bromophenol blue), and stored frozen at -30°C. The prepared sample was subjected to 7.5% SDS-polyacrylamide electrophoresis, the 7.5% SDS-polyacrylamide gel after electrophoresis was transferred to a membrane, and the transfer was confirmed with Ponceau S solution. After the transfer was confirmed, Western blot analysis employing rabbit anti-human ACE2 antibody (Abcom, ab108252) and HRP-labeled anti-rabbit IgG secondary antibody (Cell Signaling Technology, 7074S) was performed by conventional means.

As internal standards, each sample was subjected to 7.5% SDS-polyacrylamide electrophoresis, the 7.5% SDS-polyacrylamide gel after electrophoresis was transferred to a membrane, and the transfer was confirmed with Ponceau S solution. After the transfer was confirmed, Western blot analysis employing mouse anti-human actin antibody (MP biomedicals, 691001) and HRP-labeled anti-mouse IgG secondary antibody (Cell Signaling Technology, 7076S) was performed by conventional means.

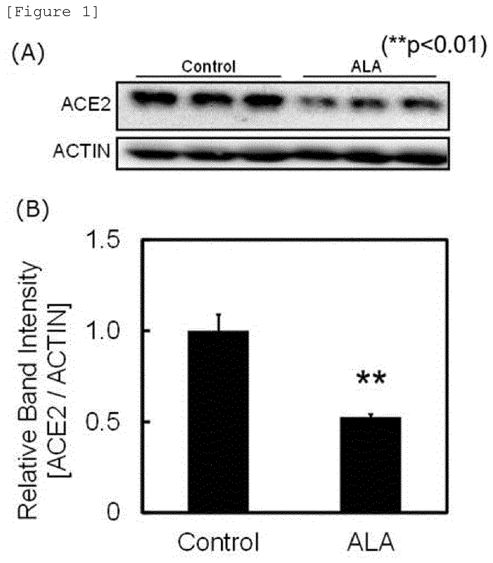

The result of Western blot analysis is shown in Figure 1 (band image: A, quantification amount: B). Note that the vertical axis of Figure 1B shows the band strength of the ALA administration group when the band strength of the control group is set as 1. As a result, compared to the control (untreated), the amount of ACE2 protein expressed was significantly reduced in HepG2 cells treated with ALA.

### [Experiment 2: Effect of 5-ALA + SFC on amount of ACE2 protein expressed]

In addition to the sample prepared in (ii) of Experiment 1, a sample having sodium ferrous citrate (SFC) added to the HepG2 cells to a final concentration of 0.5 mM, as well as samples having ALA and SFC respectively added to the HepG2 cells to a final concentration of 1 mM and 0.5 mM were employed to perform Western blot analysis similar to Experiment 1.

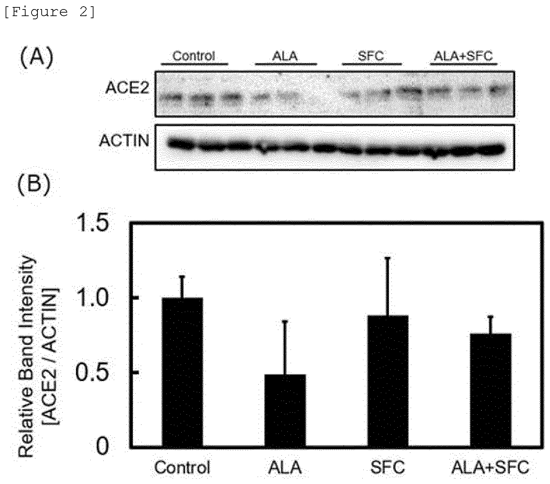

The result of Western blot analysis is shown in Figure 2 (band image: A, quantification amount: B). Note that the vertical axis of Figure 2B shows the band strength of the ALA administration group when the band strength of the control group is set as 1. Similarly to the result of Experiment 1, compared to the control (untreated), the amount of ACE2 protein expressed was reduced in HepG2 cells treated with ALA. Moreover, in HepG2 cells treated with ALA and SFC, there was also a tendency for reduction in the amount of ACE2 protein expressed. On the other hand, in HepG2 cells treated only with SFC, no reduction in the amount of ACE2 protein expressed was seen.

### [Experiment 3: In vivo change in gene expression upon oral ingestion of 5-ALA + SFC (300 mg/body)]

5-ALA (300 mg/body) + SFC (334 mg/body) was orally administered to the test subject. Using a swab kit for viral reagent transportation, nasal mucosa from the nasopharynx of the test subject was rubbed off at regular intervals, the swab was suspended in the preservation solution, and swab recovery solution was collected.

To the swab recovery solution at each collection time was added an equal amount of 70% ethanol solution, this was mixed well, and a part of the solution 1400 µL was used to perform RNA extraction according to the protocol of RNeasy Micro Kit (QIAGEN, 74004).

Eleven microliters of the extracted RNA solution were subjected to reverse transcription with SuperScript (TM) IV First-Strand Synthesis System (Thermo Fisher Scientific, 11756050) according to protocol. Fifty micromolars of Oligo d (T) 20 primer was used as the primer for reverse transcription. Since RNA extracted from the swab recovery solution can contain RNA of dead cells or bacteria, Oligo d (T) 20 primer was used with the purpose to reverse transcript only mRNA of live human cells.

The analysis subjects were ACE2 and TMPRSS2 genes, and TaqMan (TM) probe for ACTB from Thermo Fisher Scientific was used as endogenous control. Using 2 µL of the synthesized cDNA as the template, the subject gene and the endogenous control were each subjected to analysis by PCR according to protocol within the same tube with TaqMan (TM) Fast Advanced Master Mix (Thermo Fisher Scientific, 4444557).

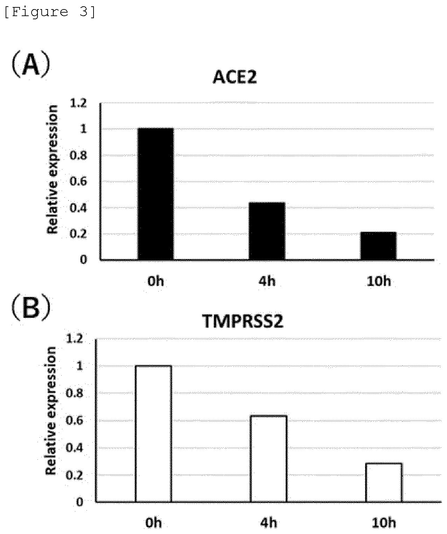

The result of analysis by PCR is shown in Figure 3 (A: amount of ACE2 expression, B: amount of TMPRSS2 expression) . Note that the vertical axis in Figure 3 shows the ratio of the amount of expression at each time, with the amount of expression of the sample at time 0 before 5-ALA + SFC ingestion (sample without 5-ALA + SFC ingestion) set as 1. The horizontal axis shows the time after 5-ALA + SFC ingestion. As shown in Figures 3A and B, after 5-ALA + SFC ingestion, the amount of ACE2 and TMPRSS2 expression in the nasal mucosa were both reduced.

### [Experiment 4: In vivo change in gene expression upon oral ingestion of 5-ALA + SFC (100 mg/body)]

5-ALA (100 mg/body) + SFC (115 mg/body) was orally administered to the test subject, and swab recovery solution was collected similarly to Experiment 3. The collected swab recovery solution was subjected to analysis by the same PCR as Experiment 3.

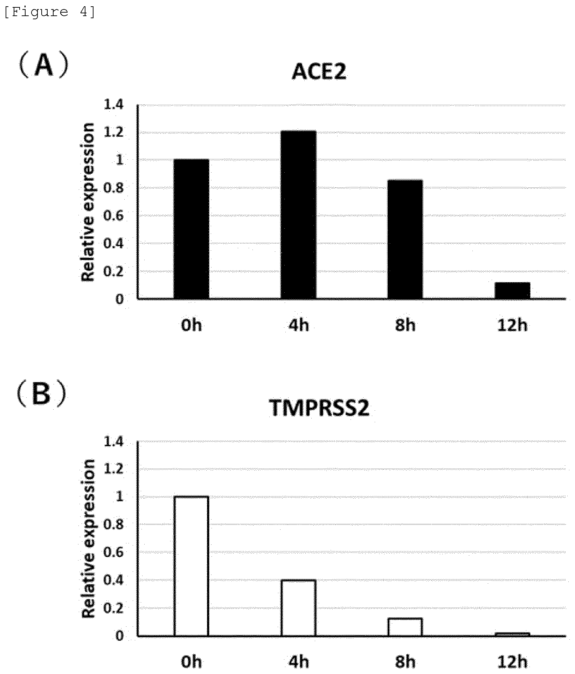

The result of analysis by PCR is shown in Figure 4 (A: amount of ACE2 expression, B: amount of TMPRSS2 expression) . Note that the vertical axis in Figure 4 shows the ratio of the amount of expression at each time, with the amount of expression of the sample at time 0 before 5-ALA + SFC ingestion (sample without 5-ALA + SFC ingestion) set as 1. The horizontal axis shows the time after 5-ALA + SFC ingestion. As shown in Figures 4A and B, after 5-ALA + SFC ingestion, the amount of ACE2 and TMPRSS2 expression in the nasal mucosa were both reduced.

## Claims

1. A pharmaceutical composition for suppressing the expression of type II transmembrane serine protease (TMPRSS2), comprising a compound shown by the following Formula (I):
[Chemical Formula 1]
R¹-NHCH₂COCH₂CH2COOR² (I)
(wherein R¹ represents a hydrogen atom or an acyl group, and R² represents a hydrogen atom, a linear or branched alkyl group, a cycloalkyl group, an aryl group, or an aralkyl group)
or a salt or an ester thereof.

2. The pharmaceutical composition according to claim 1 for further suppressing the expression of angiotensin converting enzyme 2 (ACE2) in addition to said TMPRSS2.

3. The pharmaceutical composition according to claim 1 or 2, **characterized in that**
R¹ is selected from the group consisting of a hydrogen atom, an alkanoyl group having 1-8 carbons, and an aroyl group having 7 - 14 carbons, and
R² is selected from the group consisting of a hydrogen atom, a linear or branched alkyl group having 1-8 carbons, a cycloalkyl group having 3-8 carbons, an aryl group having 6-14 carbons, and an aralkyl group having 7 - 15 carbons.

4. The pharmaceutical composition according to claim 3, **characterized in that** R¹ and R² are hydrogen atoms.

5. The pharmaceutical composition according to any one of claims 1 to 4, **characterized in that** it further contains one or two or more types of metal-containing compounds.

6. The pharmaceutical composition according to claim 5, **characterized in that** the metal-containing compound is a compound containing iron, magnesium, zinc, nickel, vanadium, copper, chromium, molybdenum, or cobalt.

7. The pharmaceutical composition according to claim 6, **characterized in that** the metal-containing compound is a compound containing iron.

8. The pharmaceutical composition according to claim 7, **characterized in that** said compound containing iron is sodium ferrous citrate.

9. The pharmaceutical composition according to any one of claims 1 - 8 for use in prevention or treatment of a disease related to expression of TMPRSS2 or ACE2 in a cell or tissue.

10. The pharmaceutical composition according to claim 9, wherein said disease related to expression of TMPRSS2 or ACE2 is an infection by a virus that infects by utilizing TMPRSS2 or ACE2.

11. The use of a compound shown by the following Formula (I):
[Chemical Formula 2]
R¹-NHCH₂COCH₂CH₂COOR² (I)
(wherein R¹ represents a hydrogen atom or an acyl group, and R² represents a hydrogen atom, a linear or branched alkyl group, a cycloalkyl group, an aryl group, or an aralkyl group)
or a salt or an ester thereof, in manufacturing a medication for suppressing the expression of type II transmembrane serine protease (TMPRSS2).

12. A method for suppressing the expression of type II transmembrane serine protease (TMPRSS2), comprising a step of applying to a subject an effective amount of a compound shown by the following Formula (I):
[Chemical Formula 3]
R¹-NHCH₂COCH₂CH₂COOR² (I)
(wherein R¹ represents a hydrogen atom or an acyl group, and R² represents a hydrogen atom, a linear or branched alkyl group, a cycloalkyl group, an aryl group, or an aralkyl group)
or a salt or an ester thereof.

13. A kit for suppressing the expression of TMPRSS2, **characterized in that** it comprises a compound shown by the following Formula (I): 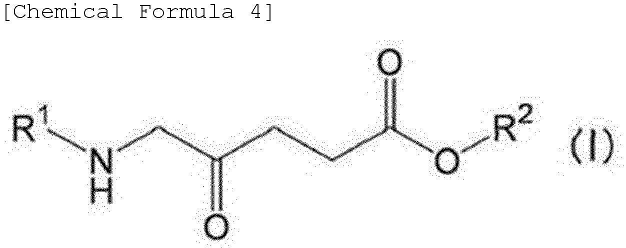 (wherein R¹ represents a hydrogen atom or an acyl group, and R² represents a hydrogen atom, a linear or branched alkyl group, a cycloalkyl group, an aryl group, or an aralkyl group)
or a pharmaceutically acceptable salt or ester thereof.
